# EUROPÄISCHE PATENTANMELDUNG

(11) **EP 4 737 466 A1**
(43) Veröffentlichungstag der Anmeldung: **06.05.2026**
(21) Anmeldenummer: 24210819.9
(22) Anmeldetag: 05.11.2024
(51) Int. Cl.: C07F 9/6574, C07C 45/50

(54) **BISPHOSPHITE MIT EINEM NAPHTHYL-FLÜGELBAUSTEIN UND EINEM PHENYL-PHENYL-FLÜGELBAUSTEIN**

(71) Anmelder: Evonik Oxeno GmbH & Co. KG, 45772 Marl (DE)
(72) Erfinder: BÜKER, Julia, 44803 Bochum (DE); FRANKE, Robert, 45772 Marl (DE); FRIDAG, Dirk, 45721 Haltern am See (DE); BILKE, Marius, 45711 Datteln (DE); BRUNS, Bastian, 44791 Bochum (DE); BÜRK, Vincent, 45665 Recklinghausen (DE); KUCMIERCZYK, Peter, 44628 Herne (DE); MARKOVIC. Ana, 45721 Haltern am See (DE); PESCHKE, Roland, 47057 Duisburg (DE); WESSNER, Maximilian, 44139 Dortmund (DE)
(74) Vertreter: Evonik Patent Association

(57) **Zusammenfassung**

Bisphosphite mit einem Naphthyl-Flügelbaustein und einem Phenyl-Phenyl-Flügelbaustein und deren Verwendung in der Hydroformylierung.

## Beschreibung

Die Erfindung betrifft Bisphosphite mit einem Naphthyl-Flügelbaustein und einem Phenyl-Phenyl-Flügelbaustein und deren Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

In WO 02/00670 A1 werden Bisphosphitverbindungen und deren Verwendung in der Olefinhydroformylierung beschrieben. Hier wird unter anderem die Verbindung **I f** gezeigt:

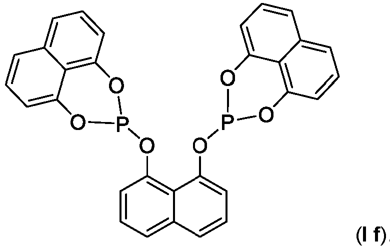

Die technische Aufgabe der Erfindung ist die Bereitstellung einer Verbindung, mit welcher bei der Hydroformylierung von Olefinen eine gute Ausbeute erzielt werden kann.

Die Aufgabe wird gelöst durch eine Verbindung gemäß Anspruch 1.

Verbindung gemäß der Formel (**I**): 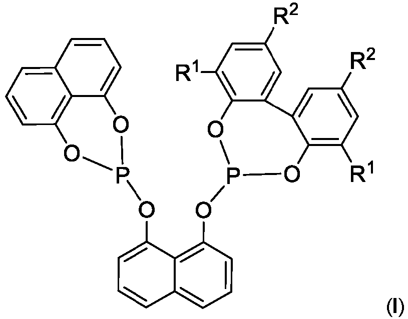 wobei R¹ und R² ausgewählt sind aus: -(C₁-C₆)-Alkyl, -O-(C₁-C₆)-Alkyl.

In einer Ausführungsform sind R¹ und R² ausgewählt aus: -(C₁-C₄)-Alkyl, -O-(C₁-C₄)-Alkyl.

In einer Ausführungsform sind R¹ und R² ausgewählt aus: -CH₃, -*^{t}*Bu, -O-CH₃.

In einer Ausführungsform steht mindestens einer der Reste R¹ oder R² für -*^{t}*Bu.

In einer Ausführungsform steht R¹ für -*^{t}*Bu.

In einer Ausführungsform R² für -*^{t}*Bu.

In einer Ausführungsform stehen R¹ und R² für den gleichen Rest.

In einer Ausführungsform weist die Verbindung die Struktur (1) auf:

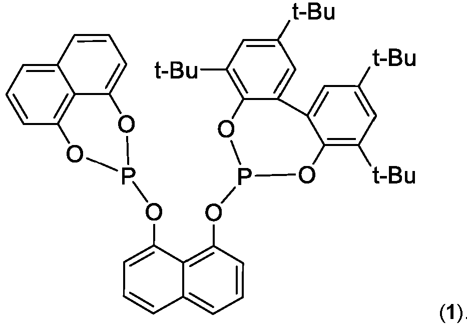

Neben den Verbindungen selbst wird auch ein Verfahren beansprucht, in dem die zuvor beschriebenen Verbindungen zum Einsatz kommen.

Verfahren umfassend die Verfahrensschritte:
a) Vorlegen eines Olefins;
b) Zugabe einer zuvor beschriebenen Verbindung;
c) Zugabe einer Substanz, welche Rh umfasst;
d) Zuführen von H₂ und CO;
e) Erwärmen des Reaktionsgemisches aus a) bis d), wobei das Olefin zu einem Aldehyd umgesetzt wird.

In einer Variante des Verfahrens ist die Substanz, welche Rh umfasst, ausgewählt aus: Rh(acac)(CO)₂, Rh(acac)(cod) (Umicore, acac = Acetylacetonat-Anion; cod = 1,5-Cyclooctadien), Rh₄CO₁₂.

In einer Variante des Verfahrens ist die Substanz, welche Rh umfasst, Rh(acac)(CO)₂.

Im Folgenden soll die Erfindung anhand eines Ausführungsbeispiels näher erläutert werden.

### Synthese

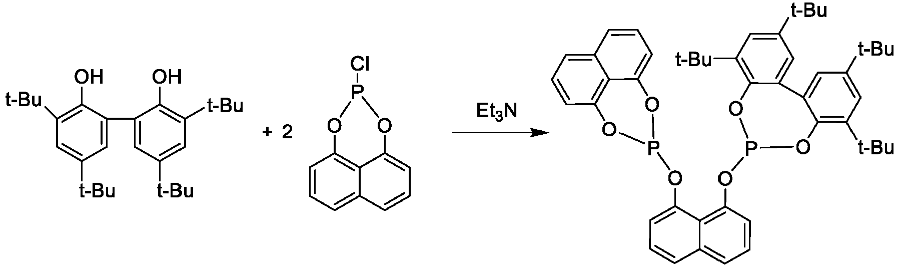

Es wurden 2 mmol 3,3',5,5'-Tetra-tert-butyl-[1,1'-biphenyl]-2,2'-diol in 10 mL getrocknetem Toluol und 5 mmol entgastem Triethylamin gelöst. Es wurden 4,4 mmol des Chlorophosphits in 10 mL getrocknetem Toluol gelöst und unter Rühren bei Raumtemperatur zu der ersten Lösung hinzugetropft. Das entstandene Ammoniumhydrochlorid wurde abgefrittet, der Filterkuchen zweimal mit je 10 mL Toluol nachgewaschen und das erhaltene Filtrat mittels Ölpumpenvakuums bei 40 °C bis zur Trockene eingeengt. Das erhaltene Produkt wurde mittels Säulenchromatographie aufgereinigt.

Ausbeute: 62%

### Katalyseversuche

Es wird unter Argon-Atmosphäre gearbeitet. Reaktionsgefäße sind zuvor unter Einwirkung von Temperatur (80 °C) und Ölpumpenvakuum getrocknet worden. Flüssige Substanzen wurden für mind. 15 Minuten durch Einperlen von Argon entgast. Die Hydroformylierung wurde in einem mit Druckkonstanthaltung ausgestatteten 0,5 L-Autoklaven der Firma Berghof Products + Instruments GmbH durchgeführt. Beheizt wird der Reaktor mittels Ölbad der Firma IKA. Der Reaktor dient zum Gasaustausch und als Temperierwerk. Innerhalb dieses Reaktors wurden 5 Glasvials (20 mL), gefüllt mit Katalysatorlösung und Magnetrührfischen unter Argon gebördelt, so platziert, dass ein Gasaustausch zwischen Vials und Reaktorraum möglich ist. Durch im Reaktor enthaltenes Thermalöl wurden die Glasvials temperiert. Angegebene Reaktionstemperaturen wurden im inneren der Glasvials gemessen. Als Substrat eingesetzt wurde n-Octen (Oxeno GmbH, Octenisomerengemisch aus 1-Octen: 3 %; cis+trans-2-Octen: 49 %; cis+trans-3-Octen: 29 %; cis+trans-4-Octen: 16 %; gerüstisomere Octene: 3 %).

Für einen Versuchslauf wurde eine Stammlösung vorab unter Argonatmosphäre vorbereitet. Hierfür wurden 0,0127 g Rh(acac)(CO)₂ und die entsprechende Menge an Phosphitverbindung (MV Lig:Rh = 5:1) eingewogen und mit 48,0 ml Toluol aufgefüllt. Von dieser Lösung wurden je ca. 8 mL auf die Vials verteilt und die genaue Menge gewogen. Die Vials wurden im Reaktor platziert und dieser verschlossen. Es wurde dreimal mit Argon und dreimal mit Synthesegas (Linde; H₂ (99,999 %) : CO (99,997%) = 1:1) gespült. Nach erfolgter Druckprüfung wurde der Autoklav bei einem Gesamtdruck von 10 bar unter Rühren (900 U/min) auf die angestrebte Temperatur von 120 °C aufgeheizt. Nach Erreichen der Reaktionstemperatur wurde der Synthesegasdruck auf 20 bar erhöht und das Substrat mittels HPLC-Pumpe mit je 2 mL zum Reaktionsstart zudosiert. Daraus ergibt sich eine Rh-Konzentration von 100 ppm. Nach 1 h Reaktionsstart bei konstantem Druck wurde eine Probe je Vial gezogen und unverdünnt gaschromatographisch analysiert.

### Ergebnisse der Katalyseversuche

Die Ergebnisse der Katalyseversuche sind in der nachfolgenden Tabelle aufgelistet

**Tabelle**

| Ligand | Ausbeute [%] |
|---|---|
| **1** | 50 |
| **I f *** | 0 |

| | |
|---|---|
| *) nichterfindungsgemäßes Vergleichsbeispiel | |

Die durchgeführten Versuche belegen, dass die gestellte Aufgabe durch eine erfindungsgemäße Verbindung gelöst wird.

## Patentansprüche

1. Verbindung gemäß der Formel (**I**): 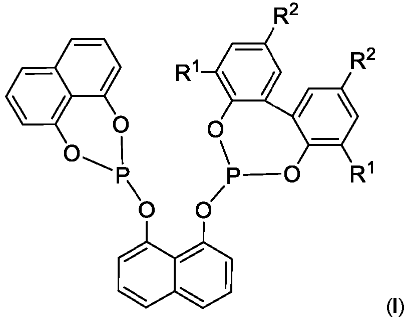 wobei R¹ und R² ausgewählt sind aus: -(C₁-C₆)-Alkyl, -O-(C₁-C₆)-Alkyl.

2. Verbindung nach Anspruch 1,
wobei R¹ und R² ausgewählt sind aus: -(C₁-C₄)-Alkyl, -O-(C₁-C₄)-Alkyl.

3. Verbindung nach einem der Ansprüche 1 oder 2,
wobei R¹ und R² ausgewählt sind aus: -CH₃, -*^{t}*Bu, -O-CH₃.

4. Verbindung nach einem der Ansprüche 1 bis 3,
wobei mindestens einer der Reste R¹ oder R² für -*^{t}*Bu steht.

5. Verbindung nach einem der Ansprüche 1 bis 4,
wobei R¹ für -*^{t}*Bu steht.

6. Verbindung nach einem der Ansprüche 1 bis 5,
wobei R² für -*^{t}*Bu steht.

7. Verbindung nach einem der Ansprüche 1 bis 6,
wobei R¹ und R² für den gleichen Rest stehen.

8. Verbindung nach einem der Ansprüche 1 bis 7,
wobei die Verbindung die Struktur (1) aufweist:

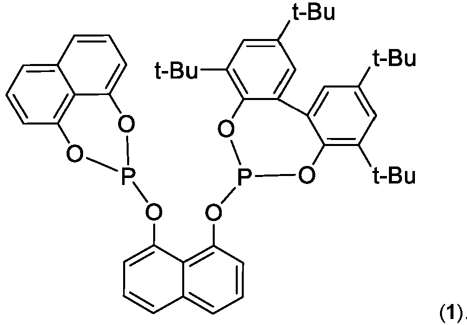

9. Verfahren umfassend die Verfahrensschritte:
a) Vorlegen eines Olefins;
b) Zugabe einer Verbindung nach einem der Ansprüche 1 bis 8;
c) Zugabe einer Substanz, welche Rh umfasst;
d) Zuführen von H₂ und CO;
e) Erwärmen des Reaktionsgemisches aus a) bis d), wobei das Olefin zu einem Aldehyd umgesetzt wird.

10. Verfahren nach Anspruch 9,
wobei die Substanz, welche Rh umfasst, ausgewählt ist aus: Rh(acac)(CO)₂, Rh(acac)(cod) (Umicore, acac = Acetylacetonat-Anion; cod = 1,5-Cyclooctadien), Rh₄CO₁₂.

11. Verfahren nach einem der Ansprüche 9 oder 10,
wobei die Substanz, welche Rh umfasst, Rh(acac)(CO)₂ ist.
